# EUROPEAN PATENT APPLICATION

(11) **EP 4 427 773 A1**
(43) Date of publication of application: **11.09.2024**
(21) Application number: 23161032.0
(22) Date of filing: 09.03.2023
(51) Int. Cl.: A61M 5/20, A61M 5/32

(54) **RESETTABLE AUTOINJECTOR**

(71) Applicant: Ypsomed AG, 3401 Burgdorf (CH)
(72) Inventor: Bosshard, Simon Martin, 3324 Hindelbank (CH); Schneider, Andreas, 3027 Bern (CH); Gerner, Sebastian, 3006 Bern (CH); Hayton, Paul Graham, Bristol, BS65AU (GB); Hill, Matthew Peter, Bangor, LL574LI (GB); Karimi, Shima, 3013 Bern (CH)
(74) Representative: Kleiner, Stefan

(57) **Abstract**

The present invention relates to an autoinj ector (1) for automated dispense of a liquid product through a needle (21) of a prefilled syringe (20). The autoinjector (1) comprises a syringe unit (2) including a syringe holder (12) a needle cover sleeve (13) and a syringe chassis (10) for supporting the syringe holder (12) and guiding a movement of the needle cover sleeve (13). The autoinjector (1) further comprises a resettable drive unit (3) comprising a plunger rod (60), a drive spring (63), a holding element (51) for holding the plunger rod (60) in the proximal position prior to a dispense activation and a drive chassis (50) for supporting the drive spring and releasably attachable to the syringe holder (12). The plunger rod (60) comprises a retaining element (65) to retain the plunger rod within the drive unit (3).

## Description

### FIELD OF THE INVENTION

The present invention relates to drug delivery devices or medicament delivery devices for injecting, delivering, administering, infusing or dispensing substances and/or liquids such as insulin, hormone preparations or vaccines. It departs from an autoinjector for automated dispense of a liquid product through a needle of a prefilled syringe. The autoinjector comprises a syringe unit including a syringe holder and a needle cover sleeve. Furthermore, the autoinjector comprises a drive unit including a plunger rod, a drive spring for emptying the entire volume of the syringe and a drive chassis releasably attachable to the syringe holder.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices may comprise an electronic dose dial mechanism to automatically set a dose.

Also known is the use of autoinjectors with prefilled syringes. Autoinjectors usually comprise a body for housing the syringe as well as an automatic drive mechanism in order to move the plunger of the syringe upon actuation of the autoinjector. The drive mechanism of the autoinjector typically comprises a source of drive, such as a motor or strong spring for moving a transfer element, for example a rod, which acts on the plunger of the syringe. The prefilled syringe has a needle or cannula that is permanently attached to a first end of a syringe barrel or reservoir that is sealed at the opposing end by the plunger.

For safety reasons it is desirable that the needle does not protrude from a housing of the autoinjector with the exception of the time when the needle is used for injection of a medicament. Thus, either the autoinjector moves the needle out of the housing for the injection and back into the housing after injection or the autoinjector provides a needle cover or needle guard which may be moved to unsheathe the needle for an injection and which may be moved back in a needle covering position after the injection.

The majority of autoinjectors are configured as single use devices which incorporate both the syringe and the drive mechanism in the same housing. Such devices are usually disposable for ease of use.

From an ecological aspects it is desirable to reduce the waste and to reuse devices as long as possible. In order to account for both ecological responsibility and medical and safety standards devices are developed as two-part or semi-disposable or semi-reusable autoinjectors. Such autoinjectors comprise a part which includes the syringe or reservoir with the drug and which can be separated and disposed of separately from the other part, for example, from a drive unit including a drive mechanism of the autoinjector.

WO 2017/033193 A1 discloses an automatic injection device including a disposable medicament module with a prefilled syringe and a reusable drive module. The drive module includes a compressible drive spring. Upon activation by the user the drug is dispensed from the medicament module by means of the drive spring of the drive module. If the medicament module is removed from the drive module manually by a user the plunger rod is held by a retaining element within a housing of the drive module. The retaining element engages the plunger rod in a middle portion of the plunger rod. The retaining element is movable relative to the housing and can engage the housing by holding arms.

### DESCRIPTION OF THE INVENTION

It is an objective of the invention to provide a reliable dispense drive resetting mechanism of a resettable autoinjector.

This objective is achieved by the autoinjector or the method according to the independent claims. Preferred embodiments are evident from the dependent claims.

The invention relates to an autoinjector for automated dispense of a liquid product (drug or medication) through a needle of a prefilled syringe (PFS) of the autoinjector. The autoinjector includes a syringe unit and a resettable drive unit releasably attachable to the syringe unit. The syringe unit includes
- a syringe holder for holding the prefilled syringe and preferably the syringe holder is adapted to support a distal shoulder of the syringe;
- a needle cover sleeve for shielding the needle in an extended or distal end position of the needle cover sleeve;
- a syringe chassis for mechanically supporting the syringe holder and guiding a movement of the needle cover sleeve from the extended position to a retracted or proximal end position of the needle cover sleeve in which the needle is exposed.

The resettable drive unit comprises
- a plunger rod moveable from a proximal position to a distal position of the plunger rod for driving a stopper in a barrel of the prefilled syringe towards the needle to dispense the liquid product;
- a mechanical drive spring for emptying the entire volume of the barrel in a single dispensing event;
- a holding element for holding the plunger rod or the drive spring in a pretensioned state prior to dispense activation;
- a drive chassis for mechanically supporting the drive spring and for supporting further internal components of the drive unit. The drive chassis is releasably attached or attachable to the syringe chassis for attachment of the syringe unit to the drive unit.

The plunger rod comprises a retaining element to retain the plunger rod within the drive unit upon separation of the drive unit and the syringe unit after product dispense. The retaining element is provided at a proximal end, in a proximal end portion or in a proximal half of the plunger rod. In an initial state and in a dispensed state the retaining element is located proximally of a distal end of the drive spring.

After a tool-wise separation of the autoinjector into the syringe unit and the drive unit the plunger rod is no longer guided inside the barrel of the syringe or by elements of the syringe unit. In such a detached state the retaining element reliably prevents the plunger rod from separating from the drive unit and in particular from separating from the drive chassis. As the retaining element is arranged at a proximal end of the plunger rod a compact and space-saving design is provided. Furthermore, due to the proximally arranged retaining element the plunger rod does not have to be retracted from a distal position to a proximal position prior separation of the syringe unit and drive unit.

The retaining element holds the plunger rod to the drive unit (in particluar after separation of the syringe unit and drive unit) but allows the plunger rod to move relative to the drive chassis. Namely, the plunger rod may be movable rotationally or linearly relative to the drive chassis. The retaining element may be, for example, a knob, a protrusion, recess, hook or a thread on the plunger rod. The drive chassis or any other component of the drive unit preferably includes a counter element with a counter stop surface, engaging the retaining element in a distal position of the plunger rod. Such a counter element may be, for example, a cam, slot, protrusion or recess.

The retaining element includes preferably a distal stop surface adapted to engage a proximal counter stop surface of a drive chassis component to retain the plunge rod. The counter stop surface may be rigid or elastically mounted a component of the drive unit to engage the distal stop surface to reliably hold the plunger rod when the syringe unit is separated from the drive unit.

The autoinjector according to the invention is resettable. That means after use the autoinjector can be returned to a manufacturer or reprocessing site to reset and prepare the autoinjector for reuse. To this end, the autoinjector is separated into the syringe unit and the drive unit. A dedicated tool may be required for disassembly of the autoinjector and for separation of the drive unit and the syringe unit.

The syringe unit and the drive unit are then subject to function tests and in particular a drive spring test may be carried out to assess whether the drive spring fulfills predefined requirements such as minimal drive force. In a further step the syringe unit and drive unit may be industrially cleaned or sterilized. If the drive spring test is passed and if the drive spring fulfills the requirements it is reset, for example a mechanical spring is tensioned or an elastic material is compressed to prepare the drive unit for a reuse.

During these preparation steps the retaining element reliably holds the plunger rod within the drive unit and thus avoids a detachment of the plunger rod. This provides for an efficient handling and assembly of the drive unit during the reset procedure.

After loading the syringe unit with a new prefilled syringe the syringe unit and drive unit are reconnected. After quality a check the autoinjector is ready for use for a new injection.

In contrast to known semi-reusable autoinjector concepts with user-manipulated syringe cassettes the syringe unit of the autoinjector according to the invention is not completely discarded but only the emptied syringe is replaced. The syringe chassis can be reused and reconnected to the drive chassis once a new prefilled syringe is loaded. That reduces waste compared to semi-reusable autoinjectors with syringe units that are entirely disposed of after use.

Besides that, the invention provides an autoinjector drive unit that can be reset or re-charged, preferably manually by the manufacturer or a person at the reprocessing site and thus allows also a reuse of the drive unit. The resettable autoinjector according to the invention contributes thus to the reduction of the carbon footprint per injection.

The drive unit preferably comprises a mechanical drive spring, preferably a helix-type, compression spring, which is preferably manually resettable and re-chargeable.

The drive unit or the syringe unit preferably includes further a lockout element for locking the needle cover sleeve in the extended position against proximal movement with respect to the syringe chassis. That means after the injection when the user has removed the autoinjector from the injection site the lockout element locks the needle cover sleeve in the extended or distal position to cover the needle and to prevent injuries. During the reset procedure the lockout element is preferably reset too to allow the needle cover to be pushed again in proximal direction during a new injection.

The drive unit further includes a holding element for holding the plunger rod in the prestressed proximal position. The holding element is released upon activation, preferably upon retraction of the cover sleeve from the extended position into the retracted position, such that the plunger rod is free to move in distal direction to dispense the liquid product. Examples for a holding element are an elastic or/and flexible arm, a snap element or a movable cam or stopper.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the autoinjector carrying the injection needle or an injection cannula, whereas the term "proximal" is meant to refer to the opposite direction or end pointing away from the needle or cannula.

The term "injection system", "injector" or "autoinjector" refers to a device that is removed from the injection site after each medication event or drug delivery process. In contrast, an infusion system refers to a device with a cannula or needle that remains in the skin of the patient for a prolonged period of time, for example, several hours.

In a preferred embodiment the retaining element on the plunger rod is a radially extending protrusion or a recess. The drive chassis or any component of the drive unit comprises preferably a counter element adapted to engage the protrusion or recess to retain the plunger rod. The protrusion or recess on the plunger rod and the counter element thus form a form-fit connection. Examples for a counter element are an indentation, slot, stopper, hook or a counter protrusion.

The form-fit connection provides for a simple and reliable engagement of the plunger rod with the chassis or any other drive unit component. Although the plunger rod is held by the connection between the protrusion or recess and the counter element in a distal end position of the plunger rod the latte is movable relative to drive chassis but is prevented from being expelled from the drive unit.

Alternatively, the retaining element may be a thread coupling the plunger rod with the drive chassis or with any other drive unit component.

Preferably, the autoinjector includes an outer shroud extending longitudinally between a distal and proximal end and covering at least partially the syringe unit and the drive unit in an attached state. The shroud thus envelops the drive unit and the syringe unit and thus provides an outermost cover for the syringe unit and the drive unit. In a preferred embodiment the shroud is sleeve-shaped extending along the longitudinal axis of the syringe unit and the drive unit.

The shroud preferably does not support or guide inner elements or components and thus does not hold a drive mechanism or the syringe. The shroud is thus not part of a supporting housing. Instead, the shroud is exclusively attached outside the drive chassis and syringe chassis.

The drive chassis and the syringe chassis provide a supporting structure for the autoinjector components but the drive chassis and syringe chassis do not envelope or cover the component and are not to be interpreted as a housing either. The drive chassis and the syringe chassis are preferably a mere support structure and include several openings and breakouts which allow direct access to the components. The internal components are preferably shielded and covered exclusively by the mounted shroud.

In an assembled state the syringe unit and the drive unit are arranged coaxially inside the shroud. The shroud is preferably tool-wise connected to at least one of the syringe unit and the drive unit, preferably by a snap-fit connection between the shroud and the syringe chassis or the drive chasses. In a preferred embodiment the shroud may be disassembled exclusively by a dedicated tool and a person at the reprocessing site (e. g. not by the user). That means the user cannot disassemble the autoinjector and therefore unintentional use or injuries can be prevented.

The shroud allows for customizing the autoinjector by providing specific visual elements (labels, brands, non-electronic or electronic identifiers such as a RFID tag) or by providing a dedicated outer form or geometry, for example, a user gripping portion.

The shroud is preferably disposable. That means the shroud is discarded during the reset procedure of the autoinjector. Accordingly, a new shroud may be mounted during the reset of the autoinjector.

The shroud is preferably made of a biopolymer based on non-fossil raw material. Biopolymers are a sustainable alternative to fossil-based plastics. Biopolymers are to be understood as a plastic based on biomass and not derived from petroleum-based polymers or natural gas.

Biopolymers may be obtained either by processing directly from natural biopolymers and proteins or by chemically obtained sugar derivatives, lipids or biologically generated by fermentation of sugars or lipids. Examples of biopolymers include sugar-based, starch-based or cellulose-based polymers.

Biopolymers can be biodegradable or non-biodegradable. Examples for the latter are polypropylene (bio-PP), polyethylene (bio-PE), a bio-polyester such as polyethylene terephthalate (bio-PET), polyethylene 2,5-furandicarboxylate (bio-PEF), polyamide (bio-PA), polyoxymethylene (bio-POM), polycarbonate (bio-PC), poly acrylonitrile-butadiene-styrene copolymer (bio-ABS), thermoplastic elastomer (bio-TPE).

In a preferred embodiment the shroud is made of biodegradable polymers. The term "biodegradable" means that the plastic can be broken down by the action of living organism, in particular microbes which consume, metabolize and convert biodegradables plastics into energy and non-plastic waste products.

Biodegradable polymers include biopolymers (for example, polylactic acid PLA, starch blends, lignin-based polymer composites) but also biodegradable petroleum-based polymers (for example, polyglycolic acid PGA, polybutylene succinate PBS, polycaprolactone PCL).

In order to further reduce waste and to account for ecological demands the shroud may be made of recycled polymers. The use of recycled polymers lowers the use of new raw materials and closes the recycling loop. Recycled polymers may include biopolymers but also non-biopolymers.

As an alternative to polymers the shroud may be made of recyclable cardboard or wooden veneer. A shroud made of wooden veneer includes thin slices or thin layers of wood and/or bark that are bonded or glued together in pieces to produce the sleeve-shaped shroud. A shroud made of wood veneer benefits a low impact on total energy use, greenhouse gases, air and water pollution compared to a shroud made of plastics.

If the shroud is made of any kind of plastic the shroud is preferably manufactured by means of a one component injection molding or more component injection molding. The injection molding allows an efficient and cost-saving manufacturing for large quantities.

In a preferred embodiment the syringe holder is adapted to hold the syringe fixedly and non-movable in a longitudinal direction of the syringe relative to the drive chassis. For that purpose, the syringe holder may include stop or contact surface supporting a distal shoulder of the syringe barrel.

That means the syringe is not displaced during the injection process. This is in contrast to autoinjectors providing for a syringe movement, for example, to displace the syringe in distal direction to automatically insert the injection needle into the injection site.

Besides that, the syringe chassis is adapted to mechanically support the syringe holder and to guide a movement of the needle cover sleeve from an extended (distal) position to a retracted (proximal) position and vice versa. The needle cover sleeve is preferably biased by a needle cover sleeve spring towards the extended position.

The needle cover sleeve may trigger an activation of the drive spring. In particular, when the needle cover sleeve has reached a retracted or proximal end position the drive spring may be activated to drive the plunger rod to dispense the liquid product.

In a preferred embodiment the drive chassis or the syringe chassis includes a snapper and the other of the drive chassis or the syringe chassis includes a recess or counter element adapted to engage the snapper to form a snap-fit connection between the syringe unit and the drive unit. Examples for a counter element are an indentation, a protrusion or a hook-shaped element.

Therefore, the syringe unit and the drive unit can reliably be connected to each other. The snap-fit connection may include one or more snapper and correspondingly one or more counter element.

In a preferred embodiment the snap-fit connection is releasable. That means the syringe chassis and the drive chassis can be disconnected and reconnected several times allowing to refill and reuse the autoinjector several times. In a preferred embodiment the snap-fit connection is exclusively disconnectable by a tool.

In a further preferred embodiment the snap-fit connection between the syringe chassis and the drive chassis is exclusively accessible after removal of the shroud. The shroud extends longitudinally between a distal and proximal end of the autoinjector and covers at least partially the syringe unit and the drive unit as described above.

The snapper comprises preferably a release surface wherein the snapper is radially deflectable by pushing against the release surface thereby releasing and disconnecting the snap-fit connection between the syringe unit and the drive unit. That means the snapper can be deflected by pushing against the release surface and thus the snapper disengages from the indentation or counter element.

A device specific tool or dedicated key may be required to reach the release surface and to deflect the snapper via release surface. This avoids unintentional deflection and thus an unintentional disconnection of the syringe unit from the drive unit. In particular, the snap-fit connection may be designed such that the connection can exclusively disconnected at a manufacturer site or reprocessing site by a autoinjector specific disassembly tool.

Additionally to or instead of the tool-specific connection the release surface may be covered by a removable cover member to prevent unintentional access and release of the snapper. The cover may be implemented in form of a cover sheet or sleeve-shaped member.

In a preferred embodiment the drive unit comprises a lock member which is movable relative to the drive chassis. In a first position of the lock member a retraction of plunger rod from a distal position to a proximal position (e. g. a reset movement) is prevented by the lock member and in second position of the lock member different form the first position of the lock member the plunger rod is free to be moved back from the distal position to the proximal position (to reset the drive unit).

Hence, the lock member locks and unlocks a reset movement of the plunger rod. The first position is preferably a proximal position of the lock member and accordingly the second position is preferably a distal position of the lock member. Alternatively, the first position of the lock member is a distal position and the second position is a proximal position.

In the first position the plunger rod is preferably prevented from being moved back in proximal direction or in a retracted position. This may be achieved, for example, by an element of the lock member or an intermediate member (for example a saw tooth) which abuts the plunger rod and thus blocks a plunger rod movement towards the retracted position. In the second position the element preferably does not hold the plunger rod and the plunger rod is thus free to be pushed back in the retracted position. Hence, in the second position of the lock member the travel path of the plunger rod is free and thus a movement of the plunger rod is allowed.

Preferably, the plunger rod is hollow and the drive spring is at least partly arranged inside the hollow plunger rod. The spring is prestressed before the injection and upon activation by the user the spring is released to move the plunger rod distally in dispensing direction to dispense the product form the syringe. As the spring is arranged inside the hollow plunger rod a compact and space-saving design is provided. In a preferred embodiment the spring has coils wound around the longitudinal axis of the autoinjector.

The spring may be adapted to move the plunger rod linearly without rotation. Alternatively, the drive spring may be a prestressed spiral spring which upon activation starts to rotate the plunger rod either directly or via a driving member that in turn is adapted to advance the plunger rod in dispensing direction.

The invention further relates to a method for resetting an autoinjector including a drive unit and a syringe unit. The latter includes a prefilled syringe for automated dispense of a liquid product through a needle of the prefilled syringe The method comprising the steps of
a) Disconnecting, preferably by a device specific tool, a connection (preferably a snap-fit) between the syringe unit and the drive unit of a used autoinjector after dispensing the liquid product and separating, by a person at a reprocessing site, the autoinjector into the syringe unit and the drive unit, wherein a plunger rod is retained within the drive unit by a retaining element provided at a proximal end of the plunger rod and wherein the retaining element is located proximally of a distal end of a mechanical drive spring;
b) Removing, by a specialist at a reprocessing site or automatically, an emptied syringe from the syringe unit and preferably reloading a new syringe into the syringe unit;
c) Resetting a plunger rod of the drive unit from a distal position to a proximal position and thereby pretensioning a mechanical spring of the drive unit.

In a preferred embodiment the method may further include the step of removing an outer shroud covering the syringe unit and the drive unit before disconnecting the syringe unit and the drive unit.

Furthermore, the method includes preferably the step of loading a new syringe into the syringe unit after the emptied syringe was removed and reconnecting the syringe unit and the drive unit after the plunger rod was reset.

Besides that, a cover lock may be reset in the syringe unit. The cover lock locks the cover sleeve in an extended position in which the cover sleeve covers the needle after an injection. The reset of the cover lock releases the cover sleeve and allows cover sleeve to be moved from an extended position into a retracted position in which the needle is exposed and not covered by the cover sleeve. Preferably, the method further comprises the step of industrial cleaning the drive unit after disconnecting from the syringe unit. The drive unit may be, for example, washed or heat treated to prepare the drive unit for a use with a new syringe unit including a new prefilled syringe.

The method may further include the step of moving a lock sleeve in a release position to allow reset of the plunger rod to the proximal position and subsequently moving the lock sleeve in a lock position to prevent movement of the plunger rod in the distal direction before the plunger rod is released for an injection.

Furthermore, the method comprises preferably the step of connecting a new shroud to the syringe unit, wherein the shroud at least partially covers the syringe unit and the drive unit.

### BRIEF DESCRIPTION OF THE DRAWINGS

The subject matter of the invention will be explained in more detail in the following text with reference to preferred exemplary embodiments which are illustrated in the attached drawings, in which:
- Fig.1: depicts a schematical and sectional view of the autoinjector according to the invention wherein the cut for the sectional view runs along the longitudinal axis;
- Fig.2: depicts a schematical and sectional view of a syringe unit of the autoinjector;
- Fig.3: depicts a schematical and sectional view of a drive unit of the autoinjector in an unused state and
- Fig.4: depicts a schematical and sectional view of the drive unit in a dispensed state.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

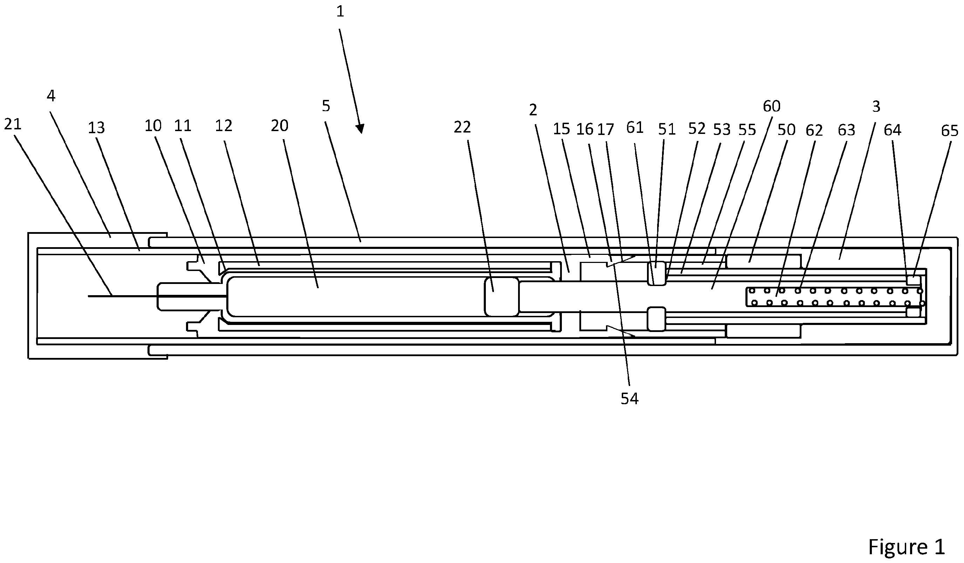

In the following the term "distal" refers to the side where an injection needle is located (e.g. left hand side in figures 1 to 4). The term "proximal" refers to the opposite side which is furthest away from the needle (right hand side in figures 1 to 4).

Figure 1 depicts a schematical and sectional view of a resettable autoinjector 1 according to the invention. The cut for the sectional view runs along the longitudinal axis of the autoinjector 1. Figure 1 depicts the autoinjector 1 in an initial or a shipping state.

The autoinjector 1 is cylindrically shaped and comprises a syringe unit 2 which accommodates a prefilled syringe (PFS) 20 containing a liquid drug and a stopper 22. The autoinjector 1 further includes a drive unit 3 which accommodates a mechanical drive spring 63 for dispensing the drug from the syringe 20. The autoinjector 1 further includes an outer shroud 5 covering and enclosing both the syringe unit 2 and the drive unit 3. In the initial state as shown in figure 1 a cap 4 is mounted on a distal end of the autoinjector 1.

The syringe unit 2 and drive unit 3 are not intended to be disconnected by the user. However, after use and return to a manufacturer or reprocessing site the autoinjector 1 can be separated by a specialist or automatically into the syringe unit 2 and the drive unit 3. The autoinjector can then be refilled by installing a new syringe and reset for a reissue and for reuse. The syringe unit 2 and drive unit 3 are described in detail in the following. Subsequently, the reset procedure of the autoinjector will be described.

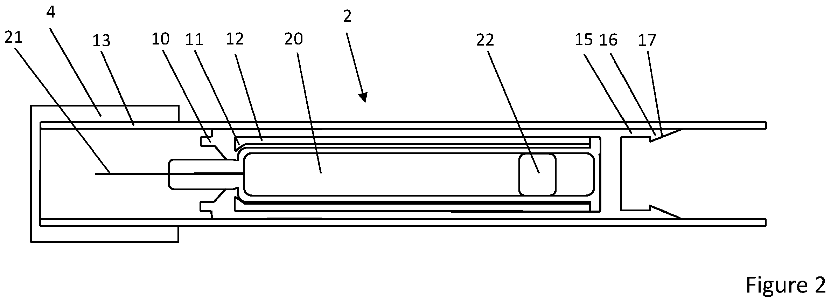

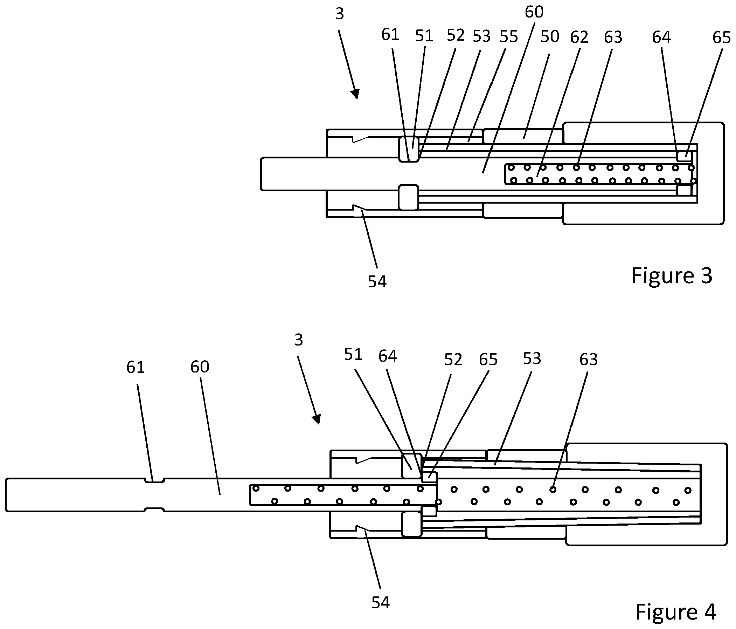

Figures 2 depicts a schematical view of a separate syringe unit 2 of figure 1 in an initial or shipping state and figure 3 depicts a schematical view of a separate drive unit 3. Both units are shown without the covering shroud 5.

As shown in figure 2 the syringe unit 2 includes a sleeve-shaped syringe chassis 10 and a syringe holder 12 supported and held by the syringe chassis 10. The syringe holder 12 in turn holds the prefilled syringe 20 non-movable relative to the chassis in place. For this purpose, a distal shoulder of the syringe barrel is supported by a support surface 11 of the syringe holder 12. The syringe chassis 10 further supports and guides a needle cover sleeve 13 which is coaxially arranged outside the syringe chassis 10. The cover sleeve 13 is movable in the longitudinal direction relative to the syringe chassis 10 from a distal or extended position in which a needle 21 of the syringe 20 is covered to a proximal or retracted position in which the needle 21 is exposed. The cover sleeve 13 is biased into the extended position by a cover sleeve spring (not shown). The cover spring is arranged in the sringe unit abut could be arranged alternatively in the drive unit.

A proximal end the syringe chassis 10 comprises two oppositely arranged snapper or flexible arms 15 adapted to engage and snap into a recess 54 in a drive chassis 50 of the drive unit 3 to connect the syringe unit 2 to the drive unit 3 by a snap-fit connection. Each flexible arm 15 features a hook 16 or hook-shaped free end which fits into the recess 54. The hook 16 in turn includes a release surface 17 which allows to engage the arm 15 and to deflect the arm 15 as will be descripted in detail below. The drive unit 3 as shown in figure 3 comprises the sleeve-shaped drive chassis 50, a hollow plunger rod 60 and a prestressed helix-type, compression drive spring 63 coaxially arranged in an opening 62 of the hollow plunger rod 60. A proximal end of the spiral spring 63 is supported by a wall of the drive chassis 50 and a distal end of the spiral spring 63 abuts an inner end wall or bottom of the opening 62 of the plunger rod 60 and thus biases the plunger rod 60 in distal direction. The plunger rod 60 is held in its biased proximal position by two oppositely arranged flexible locking arms 53 which can selectively lock or release the plunger rod 60. The arms 53 can be deflected radially outwards if a coaxially arranged lock sleeve 55 is in a release position. At a free end each arm 53 includes a cam 51 adapted to engage an indentation 61 in the plunger rod 60 to hold the plunger rod 60 in place.

The lock sleeve 55 which is supported and guided by the drive chassis 50 is movable in the longitudinal direction relative to the drive chassis between the release position and a lock position of the lock sleeve. In the lock position the cams 51 are blocked by the lock sleeve 55 such that the arms 53 cannot deflect radially outwards. The plunger rod 60 is then locked in its biased proximal position. In the release position the cams 51 can deflect radially outwards and thus the arms 53 do not hold the plunger rod 60 which is then free to move in distal or dispensing direction.

At its proximal end the plunger rod 60 includes two oppositely arranged radial protrusions 65 each including a distal stop surface 64 adapted to engage a proximal stop surface 52 located on each cam 51. That means if the plunger rod 60 is in a distal end position after dispensing the proximal stop surfaces 52 of the cams 51 abut the distally oriented stop surfaces 64 of the protrusions 65. Therefore, the plunger rod 60 is held within the drive chassis 50 and cannot drop off and cannot separate from the drive unit 3. The protrusions 65 thus act as retaining elements to prevent a separation of the plunger rod 60 from the drive chassis. The protrusions 65 are located proximally of a distal end of the drive spring 63 in an initial or unused state and in the dispensed state as shown in figures 3 and figure 4.

Alternatively, a proximal stop surface is provided on the drive chassis, or by an element axially immovable with respect to the drive chassis, at an axial location close to the proximal end of the syringe.

Turning to figure 1 the syringe unit 2 and the drive unit 3 are descripted when the units are connected to each other. As shown, the flexible arms 15 of the syringe unit 2 engage the drive chassis 50 of the drive unit 3 and thus couple and hold the syringe unit 2 and drive unit 3 together. As shown in figure 1 an outermost cover is provided by the thin-walled shroud 5 covering or enveloping the syringe unit 2 and the drive unit 3. The shroud 5 includes snap elements that engage a recess in the syringe chassis to fixedly connect the shroud 5 to the syringe unit 2 (not shown). The connecting elements are covered by a label arranged on an outside of the shroud 5. The drive unit 3 is fixedly connected to the syringe unit 2 on a distal side and enveloped by the shroud 5 on a proximal side as shown in figure 1. The shroud 5 provides a mere design element for medicament labels and/or the shroud provides a user grip portion (not shown).

The shroud 5 is made of recycled biopolymer, in particular made of polypropylene (bio-PP) and produced by injection molding. In an alternative embodiment the shroud is made of a biodegradable plastic such as lignin-based polymer composites or the shroud may be made of wooden veneer.

In the following a reset procedure of the autoinjector 1 is described in detail. After use the user either returns the autoinjector 1 directly to the manufacturer site (or to an authorized reprocessing site) by a mail service or brings the autoinjector back to a sales point, e. g. a pharmacy. The packaging and the device cap are not intended for reuse and are disposed of by the user.

At the manufacturer site (or reprocessing site) a specialist carries out a reset procedure to prepare the autoinjector for reissue to consumers.

In a first step, the autoinjector is logged either by means of an electronic tag (NFC tag) or by a serial number. If the lifetime is not yet exceeded the autoinjector 1 is forwarded for resetting.

In a second step, the autoinjector 1 is disassembled. For that, the shroud 5 is removed by releasing the snap-fit connection between shroud snap arms and the syringe chassis 10 of the syringe unit 2. An autoinjector specific disassembly tool is inserted from a distal side into the cover sleeve to reach and release the snap-fit connection. The syringe unit 2 and drive unit 3 remain still connected without the shroud 5.

After the shroud 5 has been removed and discarded a disassembly tool with two arms can be inserted through an opening in an outer surface of the drive chassis 50 to reach and release the snap-fit connection between the syringe chassis 10 and the drive chassis 50. That means after insertion the two arms of the disassembly tool contact the release surfaces 17 and further movement presses the release surfaces 17 radially outwards and thereby causes the two flexible arms 15 to deflect outwards. This releases the snap-fit connection between the syringe chassis 10 and drive chassis 50 and thus the syringe unit 2 and drive unit 3 can be separated.

In a next step, the emptied syringe 20 is disposed of. In a further step the drive unit 3 is reset. To this end, the lock sleeve 55 is shifted from a proximal locking position to a distal release position (if not already in the release position). Subsequently, the plunger rod 60 is pushed back proximally into a retracted proximal end position and thereby the drive spring 63 is tensioned. When the plunger rod 60 is in its proximal end position the lock sleeve 55 is moved back into the locking position to hold the biased plunger rod 60 in place.

In a further step, the drive spring force is tested by releasing the plunger rod to determine whether the drive spring fulfills the requirements for a reissue. If the spring force does not reach a predefined threshold or if any malfunction of the drive unit 3 is detected the drive unit 3 is discarded and replaced by a new drive unit.

In a further step, the syringe unit 2 and the drive unit 3 are subject to an industrial cleaning or sterilization. Further, a new prefilled syringe 20 is inserted into the syringe holder 12 until a distal shoulder of the syringe barrel contacts the support surface 11 of the syringe holder 12. Lateral walls of the syringe holder 12 are slightly deformed and press firmly on the syringe barrel to hold the syringe 20 in place.

In a next step, the syringe unit 2 and the drive unit 3 are connected to each other. When the flexible arms 15 of the syringe chassis 10 are inserted into the drive chassis 50 along the longitudinal axis the arms 15 are deflected and their hooks 16 snap into the recesses 54 and thus connect the syringe chassis 10 to the drive chassis 50. The syringe unit 2 and the drive unit 3 are then reconnected. A needle cover lock is reset by releasing a snap element that had been preventing retraction (movement in a proximal direction) of the needle cover after the preceding dispense event. The needle cover lock may be arranged on either of the drive und and the syringe unit. That means the cover sleeve is then able to be moved from its extended position to its retracted position and thus the cover sleeve is prepared for a new injection.

The drive unit 3 may additionally include a resettable click sleeve (not shown) adapted to generate an end click signal upon completion of the dispensing stroke. In an initial or unused state the click sleeve is biased by a click sleeve spring and held in place by a cam on the plunger rod (not shown) . Once the plunger rod has reached its distal end position after the dispensing stroke the cam no longer holds the click sleeve and the click sleeve spring can release. That means the click sleeve spring moves the click sleeve proximally a short distance and the click sleeve abuts a proximal end wall of the drive unit thereby generating a click sound to the user indicating that the injection is completed. During the reset procedure the click sleeve is moved back in its distal initial position and thereby the click sleeve spring is biased. The cam of the retracted plunger rod holds the click sleeve in place.

To complete the reset a new shroud 5 is mounted. The connected syringe unit 2 and drive unit 3 are inserted into the sleeve-shaped shroud until the snap arms of the shroud snap in the indentations in the syringe chassis 10 thereby fixing the shroud 5 to the syringe unit 2 by a snap-fit. As a final assembly step a new cap 4 is mounted on a distal end of the syringe unit 2. A quality check follows the assembly procedure. Subsequently, the autoinjector 1 is ready for reissue.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

1 Autoinjector
2 Syringe unit
3 Drive unit
4 Cap
5 Shroud

10 Syringe chassis
11 support surface
12 Syringe holder
13 Cover Sleeve
15 flexible arm
16 hook
17 release surface

20 Syringe
21 Needle
22 Stopper

50 drive chassis
51 cam
52 proximal stop surface
53 locking arm
54 recess
55 lock sleeve

60 plunger rod
61 indentation
62 opening
63 drive spring
64 distal stop surface
65 protrusion

## Claims

1. Autoinjector (1) for automated dispense of a liquid product through a needle (21) of a prefilled syringe (20), the autoinjector (1) comprises a syringe unit (2) including
• a syringe holder (12) for holding the prefilled syringe (20);
• a needle cover sleeve (13) for shielding the needle (21) in an extended position of the needle cover sleeve;
• a syringe chassis (10) for supporting the syringe holder (12) and guiding a movement of the needle cover sleeve (13) from the extended position to a retracted position,
wherein the autoinjector (1) further comprises a resettable drive unit (3) attachable to the syringe unit (2), the drive unit (3) comprising
• a plunger rod (60) moveable from a proximal position to a distal position for driving a stopper (22) in a barrel of the prefilled syringe towards the needle to dispense the liquid product;
• a resettable or re-chargeable drive spring (63) for emptying the entire volume of the barrel in a single dispensing event;
• a holding element (51) for holding the plunger rod (60) in the proximal position prior to a dispense activation;
• a drive chassis (50) for supporting the drive spring and releasably attached or attachable to the syringe holder (12);
wherein the plunger rod (60) comprises a retaining element (65) to retain the plunger rod within the drive unit (3) upon separation of the syringe unit (2) and the drive unit (3) after product dispense
**characterized in that** the retaining element (65) is provided at a proximal end of the plunger rod (60) and wherein the retaining element (65) is located proximally of a distal end of the drive spring (63).

2. Autoinjector (1) according to claim 1 wherein the retaining element (65) is a radially extending protrusion or a recess and wherein the drive unit (3) comprises a counter element (52) adapted to engage the protrusion or recess after separation of the syringe unit (2) and the drive unit (3).

3. Autoinjector (1) according to claim 1 or 2, comprising a shroud (5) extending longitudinally between a distal end and a proximal end and covering at least partially the syringe unit (2) and the drive unit (3) in an attached state.

4. Autoinjector (1) according to claim 3, wherein the shroud (5) is made of a biopolymer based on non-fossil raw material.

5. Autoinjector (1) according to any of claims 1 to 4, wherein the shroud (5) is made of a biodegradable polymer.

6. Autoinjector (1) according to any of claims 1 to 5, wherein the shroud (5) is made of a recycled polymer.

7. Autoinjector (1) according to any of claims 3 to 6, wherein the shroud (5) is manufactured by means of a one or a more component injection molding.

8. Autoinjector (1) according to any of claims 1 to 7, wherein the syringe holder (12) is adapted to hold the syringe (20) non-movable relative to the drive chassis (50) in a longitudinal direction of the syringe.

9. Autoinjector (1) according to any of claims 1 to 8 wherein the syringe chassis (2) or the drive chassis (3) includes a snapper (15) and the other of the drive chassis (3) or the syringe chassis (2) includes a recess (54) adapted to engage the snapper (15) to form a snap-fit connection if the syringe unit (2) is attached to the drive unit (3).

10. Autoinjector (1) according to claim 9, wherein the snapper (15) comprises a release surface (17) and wherein the snap-fit connection can be disconnected by an autoinjector specific tool to separate the syringe unit (2) and the drive unit (3) by pushing against the release surface (17) and thereby deflect the snapper (15).

11. Autoinjector (1) according to claim 1 to 10, wherein the drive unit (3) comprises a lock member (55) movable relative to the drive chassis (50), wherein in a first position of the lock member (55) resetting the plunger rod (60) is prevented and in a second position of the lock member (55) movement of the plunger rod back into the proximal position is enabled.

12. Autoinjector (1) according to any of claim 1 to 11, wherein the plunger rod (60) is hollow and wherein the drive spring (63) is at least partly arranged inside the hollow plunger rod (60).

13. A method for resetting an autoinjector (1) including a drive unit (3) and a syringe unit (2) for a prefilled syringe (20) for automated dispense of a liquid product through a needle (21) of the prefilled syringe (20), the method comprising the steps of
a. Disconnecting a connection between the syringe unit (2) and the drive unit (3) of a used autoinjector (1) after dispensing the liquid product and separating the autoinjector into the syringe unit (2) and the drive (3) unit, wherein a plunger rod (60) is retained within the drive unit (3) by a retaining element (65) provided at a proximal end of the plunger rod (60) and wherein the retaining element (65) is located proximally of a distal end of a mechanical drive spring (63);
b. Removing an emptied syringe (20) from the syringe unit (2);
c. Resetting the plunger rod (60) of the drive unit (3) from a distal position to a proximal position of the plunger rod (60) and thereby pretensioning the drive spring (63) of the drive unit (3).

14. Method according to claim 13, further comprising the step of removing a shroud (5) at least partially covering the syringe unit (2) and the drive unit (3) before step a).

15. Method according to claim 13 further comprising the step of loading a new syringe into the syringe unit (2) after step b) and reconnecting the syringe unit (2) and the drive unit (3) after step c.)
